(19) **Europäisches Patentamt / European Patent Office / Office européen des brevets**

(11) **EP 2 531 134 B1**

(12) **EUROPEAN PATENT SPECIFICATION**

(45) Date of publication and mention of the grant of the patent:
**02.09.2020 Bulletin 2020/36**

(21) Application number: **10707687.9**

(22) Date of filing: **04.02.2010**

(51) Int Cl.:
**A61B 18/20** (2006.01)     **A61B 18/00** (2006.01)

(86) International application number:
**PCT/IT2010/000037**

(87) International publication number:
**WO 2011/096003 (11.08.2011 Gazette 2011/32)**

(54) **DEVICE FOR THE TREATMENT OF THE EPIDERMIS**

VORRICHTUNG ZUR BEHANDLUNG DER EPIDERMIS

DISPOSITIF DE TRAITEMENT DE L'ÉPIDERME

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**12.12.2012 Bulletin 2012/50**

(73) Proprietor: **EL.EN. S.p.A.**
**50041 Calenzano (Firenze) (IT)**

(72) Inventors:
- **CLEMENTI, Gabriele**
  **I-50139 Firenze (IT)**
- **MASOTTI, Leonardo**
  **I-50019 Sesto Fiorentino (FI) (IT)**
- **GALLI, Mauro**
  **I-50019 Sesto Fiorentino (FI) (IT)**
- **ZERBINATI, Nicola**
  **I-27100 Pavia (IT)**

(74) Representative: **Mannucci, Michele et al**
**Ufficio Tecnico**
**Ing. A. Mannucci S.r.l.**
**Via della Scala, 4**
**50123 Firenze (IT)**

(56) References cited:
WO-A1-2005/007003     WO-A1-2006/116141
WO-A1-2008/128175     WO-A1-2010/102255
WO-A2-2007/095183     US-B1- 7 160 287

**Description**

Technical Field

The present invention relates to a device for treating the epidermis. More in particular, the present invention relates to a device for treating the epidermis by means of an equipment comprising a laser beam of adequate wave length, so as to obtain given effects on the epidermis, such as for example the reduction of wrinkles and an overall effect of rejuvenation.

State of the Art

Medical and aesthetic treatments are increasingly spread for improving the physical appearance, for solving problems connected to skin blemishes and also for treating and solving situations of real psychological trouble resulting from the incapacity of the subject to accept his/her appearance.

Many of the various known processes, methods, and equipment, are used for treatments aiming at reducing the aging effects and therefore in particular at eliminating or reducing the formation of wrinkles on the face and on other parts of the body, such as for example the neck and the upper part of the thorax. Recently, techniques have been developed for treating the epidermis by means of laser. In many applications, a laser beam is guided in an almost uniform manner on the portion of epidermis to be treated and performs a surface ablation process, thus eliminating the upper layers of the epidermis.

The use of the laser for treating the epidermis, in particular of the face, for reducing wrinkles and other skin blemishes, is described for example in the following works: Chernoff G, Slatkine M, Zair E, Mead D., "SilkTouch: a new technology for skin resurfacing in aesthetic surgery", in J Clin Laser Med Surg. 1995 Apr;13(2):97-100; Waldorf HA, Kauvar AN, Geronemus RG; "Skin resurfacing of fine to deep rhytides using a char-free carbon dioxide laser in 47 patients.", in Dermatol Surg. 1995 Nov;21(11):940-6; David LM, Same AJ, Unger WP., "Rapid laser scanning for facial resurfacing.", in Dermatol Surg. 1995 Dec;21(12):1031-3; Lask G, Keller G, Lowe N, Gormley D., "Laser skin resurfacing with the SilkTouch flashscanner for facial rhytides.", in Dermatol Surg. 1995 Dec;21(12):1021-4.; Apfelberg DB., "Ultrapulse carbon dioxide laser with CPG scanner for full-face resurfacing for rhytids, photoaging, and acne scars", in Plast Reconstr Surg. 1997 Jun;99(7):1817-25; Apfelberg DB, Smoller B."UltraPulse carbon dioxide laser with CPG scanner for deepithelialization: clinical and histologic study", in Plast Reconstr Surg. 1997 Jun;99(7):2089-94; Raulin C, Drommer RB, Schönermark MP, Werner S., "Facial wrinkles--ultrapulsed CO2 laser: alternative or supplement to surgical face lift?", in Laryngorhinootologie. 1997 Jun;76(6):351-7; Trelles MA, Rigau J, Mellor TK, Garda L., "A clinical and histological comparison of flashscanning versus pulsed technology in carbon dioxide laser facial skin resurfacing", in Dermatol Surg. 1998 Jan;24(1):43-9; Weinstein C., "Computerized scanning erbium:YAG laser for skin resurfacing", in Dermatol Surg. 1998 Jan;24(1):83-9; Bernstein LJ, Kauvar AN, Grossman MC, Geronemus RG., "Scar resurfacing with high-energy, short-pulsed and flashscanning carbon dioxide lasers", in Dermatol Surg. 1998 Jan;24(1):101-7; Vaïsse V, Clerici T, Fusade T., "Bowen disease treated with scanned pulsed high energy CO2 laser. Follow-up of 6 cases", in Ann. Dermatol. Venereol. 2001 Nov; 128(11): 1220-4.

Recently methods have been developed, wherein the treatment of the epidermis is discontinuous, i.e. on a given region to be treated the laser is focused in discrete areas separated from each other by areas not irradiated with the laser beam. The regions irradiated by the laser beam are subjected to an ablation in substantially cylindrical volumes, spaced from each other by wide volumes, on which no treatment is performed. Methods of this type are described in Toshio Ohshiro et al, "Laser Dermatology - State of the Art", proceedings of the 7th Congress International Society for Laser Surgery and Medicine in Connection with Laser 87 Optoelectronics, ed. Springer - Verlag, 1988, pages 513 ff. The same methods are described in US patent n. 6,997,923.

In this way an attempt is made to conjugate the need for ablating the tissue, which involves a localized damage of the tissue and an erythema due to the high heating caused by the laser, with the need for a process that is as less invasive as possible. It has been thought that, by acting in limited portions of tissue, spaced from each other by wide areas absolutely not exposed to the laser beam, treatment effects (for example of reducing or eliminating wrinkles) could be achieved, similar to that obtained through a full volume or full surface treatment of the classic type, but with a lower side effect of damage on the epidermis, a lower formation of erythemas and in general a reduction of the recovery times necessary after the treatment.

Processes of this type are described for example in the following works: Fitzpatrick RE, Rostan EF, Marchell N., "Collagen tightening induced by carbon dioxide laser versus erbium: YAG laser", in Lasers Surg. Med. 2000;27(5):395-403; Hasegawa T, Matsukura T, Mizuno Y, Suga Y, Ogawa H, Ik:eda S., "Clinical trial of a laser device called fractional photothermolysis system for acne scars", in Dermatol. 2006 Sep;33(9):623-7; Rahman Z, Alam M, Dover JS., "Fractional Laser treatment for pigmentation and texture improvement", in Skin Therapy Lett. 2006 Nov;11(9):7-11; Laubach H, Chan HH, Rius F, Anderson RR, Manstein D., "Effects of skin temperature on lesion size in fractional photothermolysis", in Lasers Surg Med. 2007 Jan;39(1):14-8; Collawn SS., "Fraxel skin resurfacing", in Ann Plast Surg. 2007 Mar;58(3):237-40; Hantash BM, Bedi VP, Chan KF, Zachary CB., "Ex vivo histological characterization of a novel ablative fractional resurfacing device", in Lasers Surg Med. 2007 Feb;39(2):87-95; Hantash BM, Bedi VP, Kapadia B, Rahman Z, Jiang K, Tanner H, Chan KF., "In vivo histological evaluation of a novel ablative fractional resurfacing device", in Lasers Surg Med. 2007 Feb;39(2):96-107.

The effectiveness of these methods is doubtful. In particular, acting on too close volumes does not allow obtaining significant improvements in terms of reduction of the recovery times, whilst treating volumes too spaced out through untreated areas involves the risk of an insufficient result and therefore the need for a second intervention. WO2006116141, WO2008128175, WO2005007003 and WO2007095183 all disclose laser treatment device to treat the dermis and are considered relevant prior art for the present invention.

Summary of the Invention

To limit or overcome entirely or partially one or more of the drawbacks of the known techniques, the present invention provides for a device and a method, wherein the treatment is less invasive, as it does not provide for treated and untreated areas, but it provides for modulating in an adequate manner the energy density of the laser beam on the surface of the epidermis. In particular, according to one aspect of the present invention, it is provided for irradiating a portion of epidermis to be treated with a series of laser beams, or with at least one scanned beam, which has a particular profile of energy density, and more precisely with a greater density in the center (i.e. near the axis of the beam) and a lower energy density towards the periphery of the beam. According to the invention the energy density has, in the direction of the radius of the section of the beam, a profile similar to a Gaussian curve.

In this way it is possible to treat all the region of epidermis of interest, without leaving regions not invested by the laser beam. However, the modulation of the beam, i.e. the variation of the energy density along the radius, from the axis to the most external region of the beam, allows obtaining a differentiated effect in each portion invested by the beam. In the central area (A) of the skin surface, invested by the laser beam, the energy density is sufficient to cause the ablation of the tissue. This causes a tissue ablation in a substantially cylindrical volume below the skin surface invested by said central area of the laser. In an annular surface surrounding the central area (A), the laser beam has a substantially lower energy density, and causes, in the volume below this annular surface (volume (B) presenting a hollow conformation) a cauterization effect, i.e. an effect of hemostasis of the blood vessels and/or an effect of shrinkage of the collagen, but not an effect of laser ablation. In the present invention, outside of this area (B), below the annular surface, there is another area (C), in the shape of a hollow volume, bordering the other areas (C) due to the actions of the laser in adjacent pointings, in which the laser beam has an intensity even lower than in the inner area (B); in this area (C) there is a biostimulation by means of the laser light that facilitates the tissue regeneration of the collagen.

In this way, given a region of epidermis to be treated, the volume underneath the entire region is exposed to the laser beam, without leaving tissue volumes not hit by the energy irradiated by the laser. In this way a greater effectiveness of the treatment is achieved. However, as the ablation effect is limited to the most internal volume, the tissue damage, the erythema and the subsequent discomforts for the patient are substantially reduced, with a consequently shorter recovery time.

Furthermore, the cauterization of the tissues in the volumes (B) exposed to the portions of beam of lower intensity, relative to the axial portion of the beam, reduces the bleeding effects. In this outer volume the intensity of the laser beam, i.e. the energy density of the beam, is sufficient to cause a shrinking effect of the collagen and therefore, even if no ablation of the tissue occurs in this area, there is a substantial contribution of the laser energy to the final result of the intervention. Shrinking of the collagen, which represents an important component of the tissue, in the areas (B) and partially in the areas (C), gives to the tissue a more compact aspect immediately after the treatment, eliminating or reducing skin slackening due to aging. Furthermore, the remaining tissue in the areas (C) is subjected to the laser biostimulation action that, in the medium and long term, facilitates acceleration of the collagen regeneration; this represents a typical aspect of the treatment introduced with the technique in question.

Contrary to the most recent techniques of fractional laser treatment, wherein the laser treats discrete areas or volumes of tissue, leaving wide areas or volumes not irradiated, the present invention therefore provides for investing all the tissue with the laser, but causing on it and inside it different phenomena by modulating, or shaping in an adequate manner the profile of energy density of the beam section.

Given a portion of epidermis to be treated, this can be irradiated simultaneously by more beams, obtained for example by a single beam through particular optical systems. The various beams are for example arranged according to an adequate pattern, e.g. a matrix pattern. However, it is possible preferably to use a single beam or more than one beam, to which a scanning movement is imparted according to coordinates (for example Cartesian or polar coordinates). In some embodiments of the present invention the emission of the beam is controlled in such a manner that single beams of laser energy are "shot" in sequence in sequentially variable positions along a preset pattern, for example according to nodes of a matrix. In other embodiments it is possible to move the laser beam from a position to the other without interrupting the emission of energy, providing a sufficiently short time to pass from a treatment position to the other. In this way, the effect of the laser during the movement from an irradiation point to the other is substantially negligible relative to the effect of the beam during the phase of stopping in a given point or position of the irradiation pattern.

In any case, it is possible to provide that adjacent beams (irradiated simultaneously or sequentially through a scanning system) have superposition areas, i.e. areas in which the effect of two adjacent beams (or of three or more adjacent beams) are superposed and summed. Obviously, also depending upon the scanning operation or multiple beam operation and, in the first case, upon the scanning time, only the spatial or also the temporal superposition of the beams shall be taken into account.

In the areas of superposition, in fact, the effects of more beams which can fall on the tissue in temporal sequence, due to the scanning of the beam will be summed. In this case it should be taken into account that in a given volume surrounding the ablation area the effects of more laser beams applied in different times are summed and therefore these effects are influenced by the fact that the heat generated by a first application of the beam in a position is at least partially dispersed due to the effect of the blood circulation (and/or of the thermal convection on the surface of the epidermis) before the effect of the second beam arrives.

According to the present invention, the energy density profile of the laser beam can be such as to define, in addition to the central volume wherein the ablation of the tissue occurs and the surrounding volume wherein the cauterization and/or shrinking of the collagen occurs, a third volume wherein the energy of the laser beam is even lower and such as not to cause substantial effects of collagen shrinking and/or of cauterization or hemostasis of the vessels, but a biostimulation effect. In fact, it is known that, by irradiating a living tissue with a laser beam, it is possible to stimulate the cell differentiation and multiplication. In this way it is possible to shorten the recovery times of the patient after the intervention, as the tissue removed by ablation is replaced more quickly by new tissue. The effect of a superposition of more beams can occur in the area of intermediate energy density, wherein the laser energy is sufficient to cause the cauterization of the vessels and/or the collagen shrinking; alternatively, or in combination, this superposition of the beams can occur only in (or also in) the most external area, wherein the laser energy is only sufficient to obtain the biostimulation of the tissue.

Therefore, the object of the present invention is a device for the laser treatment of the epidermis, comprising: a source of laser energy; a focusing system for focusing the laser energy, arranged and controlled to focus the laser energy on a plurality of adjacent volumes of an area of the epidermis; wherein said laser energy is applied to each of said volumes with a laser beam having a profile of energy density variable in a cross section of said beam, in a central area of said cross section the intensity of the beam being suitable to cause an ablation of the epidermis in a central portion of the volume hit by the laser beam, and in an external annular area of said cross section the intensity of the beam being suitable to cause hemostasis, i.e. cauterization of the blood vessels and shrinking of the collagen of the epidermis in an annular portion of the volume exposed to the laser beam, said annular portion surrounding said central portion. The adjacent volumes can be also partially superposed.

In some embodiments of the present invention, the focusing system for the laser energy is arranged and controlled so as to treat adjacent volumes of the epidermis arranged according to a pattern, in which each treated volume has a center substantially positioned on the axis of the laser beam used for treating said volume, the axes of the laser beams used to treat said adjacent volumes being arranged according to a dot matrix that can be preset.

Focusing system include both a dynamic system, comprising a scanning device, to move the beam in different positions, and a system of static type, wherein an adequate optical system subdivides for example an initial beam into a plurality of adjacent beams arranged according to an adequate pattern, for example according to a matrix.

In some embodiments of the present invention, the energy density profile of the laser beam to treat said adjacent volumes and the distance between the dots of said presettable matrix are such that the full treated area of the epidermis is subjected to the action of the laser energy, in particular in the volume outside of the annular area of the section of the laser beam, wherein the intensity of the beam is suitable to cause the hemostasis of the blood vessels, the laser energy being suitable to cause a biostimulation of the tissues.

A further object of the present invention is a device for treating with a laser radiation the epidermis of a patient, comprising: a laser source; a scanning and actuating system to address a laser beam on a plurality of portions of an area of epidermis to be treated, controlled so as to actuate a laser beam in a plurality of positions inside said area of epidermis to be treated; wherein the laser beam has an energy density profile variable from the center towards the periphery of the cross section of said beam, in a first central area of said cross section the intensity of the beam being suitable to cause an ablation of the epidermis exposed to said beam, and in a second external annular area of said cross section the intensity of the beam being suitable to cause hemostasis of the blood vessels and/or a shrinking of the collagen of the epidermis in an annular portion of the epidermis exposed to said beam.

According to a further aspect, an object of the present invention is a device for treating with a laser radiation the epidermis of a patient, comprising: a laser source; a focusing system for focusing the laser energy, arranged and controlled so as to focus the laser energy on a plurality of volumes of an area of the epidermis to be treated, wherein said laser energy is applied through beams with variable energy density, which is greater in the central area and lower in the peripheral area of the cross section of the beam; the energy density of the laser beam in the central area being such as to cause an ablation in a plurality of first portions of epidermis spaced from each other in said area, surrounded by second portions of epidermis in which the laser energy causes the cauterization or hemostasis of the blood vessels and/or the shrinking of the collagen of the epidermis, outside of said second portions being defined third portions wherein the laser beam causes a biostimulation of the tissues.

According to a further aspect, the invention relates to a method for treating the epidermis of a patient, comprising: the application of laser energy in a plurality of portions of epidermis in an area of the epidermis to be treated with a distribution of energy in each portion which causes an ablation of the epidermis in the central area of said portion of epidermis and an hemostasis of the blood vessels and/or the collagen shrinking in an annular area of said portion of epidermis, surrounding said central area and outside it.

Preferably, the method also comprises the phase of biostimulating, through the laser energy, the tissue surrounding the annular area in each portion of treated epidermis.

Preferably, all the volume of epidermis in said area is treated with said laser energy. The volume of treated tissue extends from the external skin surface for a given depth inside the epidermis, according to the characteristics of the tissue and the characteristics of the laser beam. Said penetration depth will be in general different in the various areas according to the power density of the laser, for example the penetration will be deeper in the area with the greater energy density. It is understood that all the volume of the epidermis is treated when there is at least one minimum depth of penetration, such that all the volume between the external surface and this minimum penetration depth is exposed to the laser radiation. There is therefore a layer (which can have variable thickness) of tissue achieved by the laser radiation in a continuous manner, i.e. without volumes not exposed to the laser radiation.

According to a preferred embodiment of the present invention, the areas of epidermis to be treated are sequentially exposed to a laser beam controlled by a scanning system.

According to a further aspect, an object of the present invention is a method for treating the epidermis of a patient, comprising: treating with laser energy adjacent volumes of an area of epidermis, in each volume the laser energy being applied with an energy density greater in a central portion, to cause the ablation of the tissues in said central portion, with an intermediate energy density in an intermediate portion, surrounding said central portion, to cause the collagen shrinking and/or the hemostasis or cauterization of the blood vessels in said intermediate portion, and with a lower energy density in an external portion, to cause the biostimulation of the tissues in said external portion.

According to a further aspect, the present invention relates to a device and a method for treating tissues, wherein a radio frequency emission is combined with a laser radiation, i.e. to a system comprising one or more electrodes generating a radio frequency electric field, the electrodes being designed so as to propagate the radio frequency electric field in the tissues and in particular in the epidermis under treatment. These two emissions (laser and RF) can be applied in time sequence, alternatively firstly the laser and then the RF or vice versa, or they can integrally or partially overlap each other. Preferably, a handpiece is provided with a spacer formed by one or more elements constituting at the same time electrodes for applying and propagating the radio frequency field inside the treated tissue.

Radio frequency in aesthetic treatments is known per se, see for example Goldberg DJ, Fazeli A, Berlin AL. "Clinical, laboratory, and MR1 analysis of cellulite treatment with a unipolar radiofrequency device", in Dermatol Surg. 2008 Feb;34(2):204-9; or Montesi G, Calvi eri S, Balzani A, Gold MH., "Bipolar radiofrequency in the treatment of dermatologic imperfections: clinicopathological and immunohistochemical aspects", in J.Drugs Dermatol. 2007 Feb;6(2):212-5. Combined applications laser and RF, or handpieces combined for a synergic action of the two types of energy are not known. The combination of laser radiation and radio frequency allows significant advantages to be obtained. In particular, such a combination allows obtaining the following synergic effect: in the central part of the treated region, the laser causes a tissue ablation and therefore the formation of small holes in the epidermis. These allow the lines of radio frequency electric field to close faster in the skin and therefore they allow obtaining a more effective heating of the collagen by the radio frequency field with a consequent increase in the collagen shrinking effect. The effect of the radio frequency electric field is therefore more effective than that which can be obtained only with the radio frequency field. Some of the advantages which can be obtained from this combination of laser radiation and radio frequency field can be obtained also with a power distribution of the laser beam which is traditional in shape rather than Gaussian.

## Brief description of the drawings

The invention will be better understood by following the description below and the attached drawing, which shows a non-limiting practical embodiment of the present invention. More in particular:

figure 1 shows a diagram of a device which embodies the present invention;
figure 2 shows a detail of the handpiece of the device of figure 1;
figure 3 shows a curve representing the power density as a function of the distance from the beam axis in a beam with substantially circular section;

figures 4 and 5 schematically show two different ways of applying the laser energy;
figures 6 and 7 show power density curves of adjacent laser beams to treat adjacent volumes of the epidermis in two different application modes;
figure 8 shows a diagram of a scanning system for scanning the laser beam;
figure 9 shows a diagram of a system for subdividing a main laser bean into a plurality of adjacent or consecutive laser beams;
figure 10 schematically shows an improved handpiece for the laser/radio frequency combined treatment;
figure 11 shows the use of the handpiece of figure 10;
figures 12 and 13 show time graphs illustrating the combination of the laser radiation and of the radio frequency.

Detailed description of an embodiment

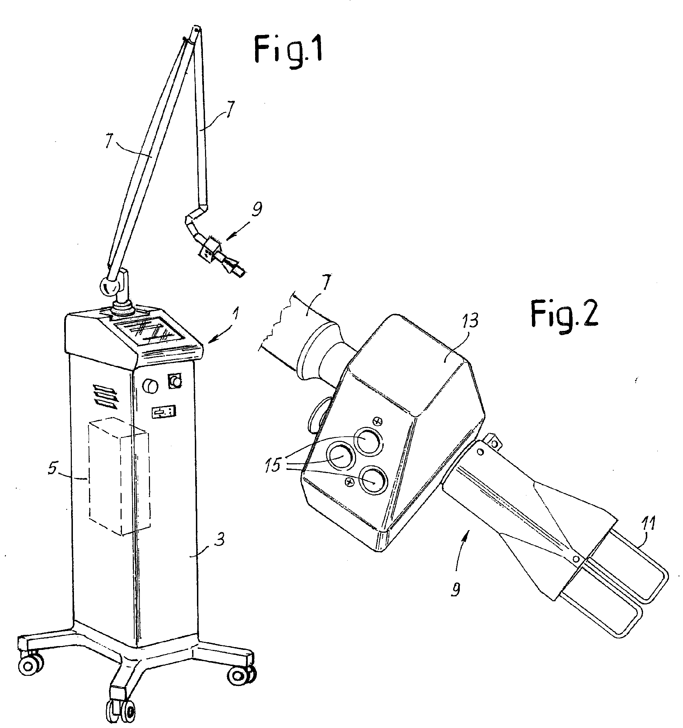

Figures 1 and 2 show a device, wherein the present invention may be embodied. In general, the device 1 comprises a base 3, inside which at least one laser source 5 is housed. The laser source 5 can be a continuous laser, but preferably a pulsed laser is used. According to some embodiments of the present invention, the laser source can have an emission wave length comprised between 532 and 13,000 nm and more in particular a wave length of 10,600 nm, corresponding to the $CO_2$ laser emission. In fact, the laser source is preferably a $CO_2$ laser.
In some embodiments of the present invention, the most relevant parameters of the equipment can fall within the following ranges of values:

| | |
|---|---|
| Type of laser: | $CO_2$, with wave length of 10.6 micrometers |
| Power irradiated to the tissue: | up to 50W |
| Repetition frequency of the pulse: | from 5 to 100 Hz |
| Duration of the pulse: | from 0.2 to 80 ms |
| Dimension of the scanning surface: | maximum 15 x 15 mm |
| Distance between two scanning dots: | up to 2 mm (step 50 micrometers or less) |

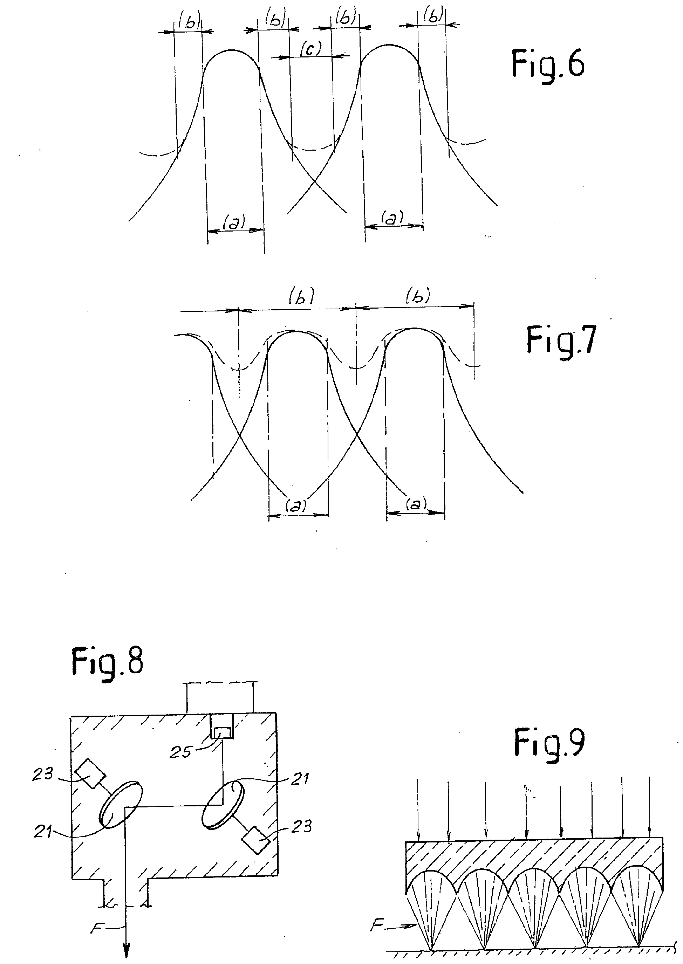

The laser can be controlled so as to provide a pulse for each position of the scanning mirrors, i.e. for each treated point. However, in other embodiments of the present invention it can be provided for "shooting" more than one laser pulse for each working position, i.e. at each treated point. For example, from two to five pulses can be provided for each position of the laser.
To obtain the Gaussian shape of the beam, the laser cavity is designed so as to insulate the main propagation mode and the focusing optical systems must be designed so as to contribute to maintaining the Gaussian shape of the energy distribution from the axis towards the outside. An adequate choice of the diameter of the cavity and an adequate radius of the mirrors of the laser source can give the generation of the oscillation mode TEM 00 that gives a Gaussian beam profile.
The laser beam can be conveyed through a wave guide 7 towards a handpiece 9. The guide can be designed in different manners, also depending upon the frequency and the emission power of the laser. In the illustrated example the wave guide is simply formed by hollow tubular elements, hinged to each other, inside which deflection mirrors for the laser beam are arranged, to deviate the beam along the axis of the various tubular portions of the guide.
Inside the handpiece 9 focusing and/or scanning systems for the laser beam are arranged, some of which are schematically represented in figures 8 and 9. Preferably, inside the handpiece 9 a scanning system (figure 8) is contained, comprising for example two scanning mirrors 21 with corresponding actuators 23 electronically controlled by a control unit, not shown. The scanning mirrors control the movement of the laser beam F exiting from the handpiece 13, so that it follows a given path, according to criteria, better defined hereunder. In this case, a single laser beam F exits therefore from the handpiece and is addressed towards the surface of the epidermis to be treated, from which the handpiece can be maintained at a constant distance, for example through a spacer 11. On the handpiece 13 buttons, grips or other regulating and interfacing members, schematically indicated with 15, can be arranged, through which the operator can modify the shape of the beam and/or the dimension and the area of the scanning surface, the movement of the beam and other.
Through the handpiece 13 and the scanning system inside it, it is possible to control the movement of the beam F according to a defined and stored pattern, which can be modified by the user if required.
A focusing optical system is arranged in an adequate point of the path of the laser beam. In the diagram of figure 8, this optical system is indicated with the number 25 and is arranged in the handpiece, but it should be understood that this is not strictly necessary, and that other positions are possible. The optical system 25 has also the function of imposing to the beam a given power density distribution according to the radius, as it will be better explained hereunder.
In other embodiments of the present invention, inside the handpiece 15 focusing systems are arranged that subdivide the laser beam into a plurality of beams that are adjacent to each other and impart an energy density profile to each of the adjacent beams, depending upon the radius according to the criteria described hereunder.

The lens inside the handpiece, combined with the shape of the beam generated by the source, causes the Gaussian profile; the generated shape of the beam depends upon the purity of the propagation mode inside the laser cavity, which determines therefore the energy distribution transversally to the axis of propagation in the free space at the exit of the laser source.

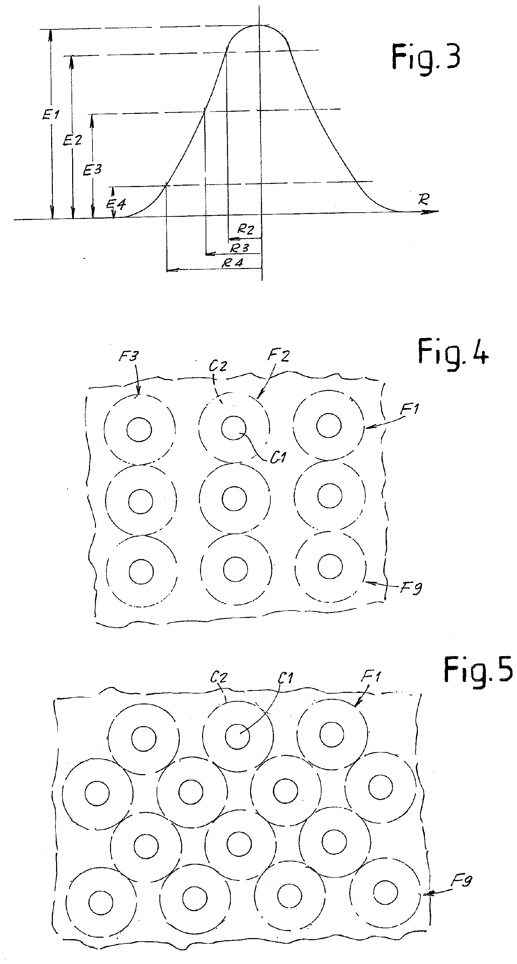

In some preferred embodiments of the present invention, the laser beam or each laser beam has a substantially circular cross section. Characteristically, the laser beam or each laser beam as a profile of energy density variable according to the distance from the axis. In other words, the energy density of the beam varies from the centre of the beam, where there is the greater density, towards the periphery of the beams, where there is the lower energy density. Figure 3 shows a curve representing the energy density, assuming to have a beam with circular cross section. In this case on the axis of ordinates the energy density is shown and on the axis of abscissas the radius is shown, i.e. the distance from the axis A of the beam. It should be noted that the energy density is maximum at the center of the beam and tends quickly to decrease moving towards the periphery of the beam. In the present invention, the energy density profile follows a trend that can be represented substantially with a Gaussian curve, as shown in figure 3. By varying the distance in the space of two contiguous pulses generated by the laser, in which there is this control (spacing of the pulses in scanning) it is possible to control the entity of the superposition of the shoulders of the radiation profiles.

On the axis of ordinates four energy density values have been identified, indicated respectively with E1, E2, E3, and E4. The values E1, E2, E3 and E4 can vary in a highly significant manner, as in their determination many parameters intervene, resulting from the interaction between laser radiation and biological tissues. These parameters depend upon the type of skin (dry or oil skin; phototype, etc.) by the angle of incidence, and by the adjustments made by the doctor based upon the experience for the various cases.

The energy density levels comprised between E1 and E2 are such that the tissue irradiated with an energy density comprised between these two levels is affected by an ablation process, i.e. a process of removal of the tissue. The energy density greater than the energy level E2 is achieved only in points contained inside the cylindrical volume of the laser beam with radius R2.

The energy density comprised between the values E2 and E3 is not sufficient to cause tissue ablation, i.e. the removal of the irradiated tissues. However, for these intermediate values of energy density cauterization phenomena occurs in the irradiated tissues, i.e. phenomena of hemostasis of the blood vessels. This avoids bleeding in the regions where the ablation occurred. Furthermore, or alternatively, the energy density levels E2 and E3 define an interval within which the laser beam causes a shrinking of the collagen contained inside the irradiated volume of tissue. Therefore in the volume irradiated with an energy density comprised between E2 and E3 a beneficial effect occurs on the collagen fibers, which causes a toning up of the tissue of the epidermis and therefore a positive result in terms of decrease of the aging effects. As in the volume irradiated with energy density comprised between E2 and E3 the ablation does not occur, the tissue damage results to be particularly limited. In figure 3, R3 indicates the radius of the cross section of the beam, inside which the energy density is greater than the level E3. Therefore, the level of energy density causing cauterization or hemostasis and/or collagen shrinking, but not tissue ablation, is achieved in an annular area of the cross section of the beam with inner radius R2 and outer radius R3.

The most external annular region of the cross section of the beam comprised between an inner radius R3 and an outer radius R4 is characterized by an energy density comprised between the level E3 and the level E4. In this interval, the laser energy is no longer sufficient to cause significant shrinking of the collagen fibers and/or cauterization or hemostasis of the blood vessels, but it is still sufficient to cause significant effects of tissue biostimulation. Consequently, in the affected area there is an effect of the laser that contributes to the post-intervention recovery, stimulating the growth of the tissues subjected to ablation in the most internal region of the portion of epidermis exposed to the single laser beam. In some embodiments of the present invention the radii R2 and R3 can assume the following values:
R2: from 60 to 175 micrometers according to the set parameters

$$R3 = R2 + 50 \text{ micrometers}$$

According to the present invention, it is advantageously provided to invest a preset area of the skin with a plurality of laser beams, each of which has an energy density shape represented qualitatively by the curve of figure 3 or anyway with a shape characterized by a gradual reduction of the energy density as the distance from the axis A-A of the beam increases. This reduction can be stepwise, instead of continuous as represented in figure 3.

The beams, with which the portion of epidermis to be treated is irradiated, can be constituted by beams arranged side by side and generated with an optical system of the type represented in figure 9, or they can be simply represented by positions assumed in time sequence by a same laser beam, moved with a scanning system as represented in figure 8. In this latter case the laser beam is preferably turned on, i.e. actuated sequentially in each desired position according to a radiation pattern, whilst during the movement between one point and the other the laser is preferably turned off.

Independently of the system of generation of adjacent laser beams, it is possible to irradiate the epidermis for example by following a pattern as represented in figure 4. Here F1, F2, F3 ... F9, represent the various laser beams in their projection on a portion of surface of the epidermis. Substantially, F1 - F9 indicate the areas defined by the intersection between the laser beam and the outer surface of the epidermis. Each region F1 - F9 has a substantially circular development if the laser beam has a circular section, but it should be understood that this is not strictly necessary, as each laser beam can have a cross section of elliptical shape or of any other adequate shape. In the example illustrated in figure A each region F1 - F9 is delimited by an inner circumference C1 and by an outer circumference C2, wherein the circumference C1 has a radius R2 and the circumference C2 has a radius R3. Therefore, inside the circumference C1 the energy density of the beam is comprised between the levels E1 and E2 as defined above with reference to figure 3, and an ablation phenomenon occurs on the surface of the epidermis and in the tissues below corresponding to the circular area C1. The depth of the tissue ablation and therefore the dimension of the affected volume depends upon various factors, among which the absorption coefficients and the power of the beam. In the circular area comprised between the circumferences C1 and C2 the laser energy density is comprised between the levels E2 and E3 and therefore in this region there is a cauterization effect of the blood vessels and/or an effect of shrinking of the collagen fibers. Outside of the circumferences C2 of each beam F1 - F9 there is an area exposed to an irradiation with a level of energy density comprised between E3 and E4 and therefore in this surface in the tissues below there is a biostimulation effect caused by the laser energy, but there is not shrinking of the collagen fibers and/or cauterization or hemostasis of the blood vessels, as well as ablation phenomena.

Figure 5 shows a different irradiation pattern. Equal numbers indicate equivalent elements to that described with reference to figure A.

The difference between the pattern of figure A and the pattern of figure 5 is simply the different position of the points in which the axis of the beam is located in the scanning, i.e. the positions in which the various beams are positioned in the case of a multi-beam irradiation system. In this case again, circular areas C1 and areas C2 with annular section can be distinguished, where there are ablation phenomena and phenomena of cauterization/shrinking of the collagen fibers respectively. In the surface portions of the epidermis outside of the circumferences C2 a biostimulation effect occurs.

Figure 6 shows qualitatively the way in which the effects of the adjacent laser beams are combined so as to obtain in the tissue of the epidermis the three phenomena of ablation, cauterization/shrinking and biostimulation. More in particular, in figure 6 two energy density distribution curves are represented for two adjacent beams. In the region indicated with A the energy density achieves the ablation values; in the regions indicated with B the energy density achieves the levels of cauterization or hemostasis of the blood vessels and/or shrinking of the collagen fibers; in the regions indicated with C, outside the regions indicated with B, the sum of the energy density of the laser beams, energy densities are achieved, sufficient for biostimulating the tissue.

It should be understood that the greater or smaller overlapping can affect the dimension of the regions subjected to shrinking of the collagen fibers/hemostasis of the vessels and/or biostimulation, depending also upon the trend of the curves representing the power density. Substantially, it is possible to model the regions subjected to the three above mentioned effects by adequately shaping the energy density profile and/or dimensioning in an adequate manner the irradiation pattern, i.e. the positions in which the single beams are arranged, with which a portion of epidermis is simultaneously irradiated, or the positions taken sequentially by a single laser with a scanning system.

In some embodiments of the present invention it is also possible to shape the energy density curves and/or to arrange the axes of the beams, with which the portion of epidermis under treatment is irradiated, so as to reduce or to completely eliminate the regions in which there is the biostimulation and to obtain in the treated volume of tissue only two effects, respectively ablation and cauterization and/or shrinking of the collagen. Such a situation is schematically represented in figure 7 with the same representing criterion of figure 6. It should be noted in figure 7 that the two beams are so close to each other as to eliminate the region indicated with C in figure 6, and therefore the tissue below irradiated by the beam will have surface regions and corresponding underneath tissue volumes subjected to ablation (regions A) and surface portions with annular development with corresponding underneath volumes inside the tissue, wherein the energy density will causes a phenomenon of cauterization/ablation and/or shrinking of the collagen fibers (regions B).

In the practical application of the method described above, a region of epidermis to be treated will be defined, and on this region the laser beams will be addressed. In a case (with a system of the type illustrated in figure 9) more beams, in case generated by a single main beam, will be addressed simultaneously on contiguous regions of the epidermis. In another case a beam will be scanned according to a pattern using a system as in figure 8. In this case the beam will be preferably interrupted in the phase of moving its axis from an irradiation point to the other. Independently of the method used, which can also combine the two systems and/or which can use two or more scanned beams, all the surface of the epidermis is irradiated, simultaneously or sequentially, but with variable energy densities. Each irradiated region will be subjected to an ablation in the central portion, to a shrinking of the collagen fibers and/or an hemostasis of the vessels in the annulus outside of the ablation region and to a biostimulation effect in the external surface.

By shaping the energy density profile and the position of the beams it is possible to modify the dimensions and the positions of these three treatment areas. The beam penetrates below the surface of the epidermis and therefore to each circular surface, annular surface or intermediate surface between the annular surfaces corresponds an underlying volume in the tissue and in the epidermis. In general, the entire surface is therefore treated and all the tissue volume within a given depth, which can vary from point to point, is irradiated by the beam, living no regions devoid of treatment, but modulating the treatment, thus obtaining different effects from a region to the other. This surprisingly allows the advantage of lower invasivity of the intervention, much faster recovery times, but at the same time an effectiveness of the treatment much greater relative to that which can be obtained with beams of substantially constant energy density in all the section and spaced from each other so as to leave regions of epidermis not irradiated and therefore where the laser has no effect.

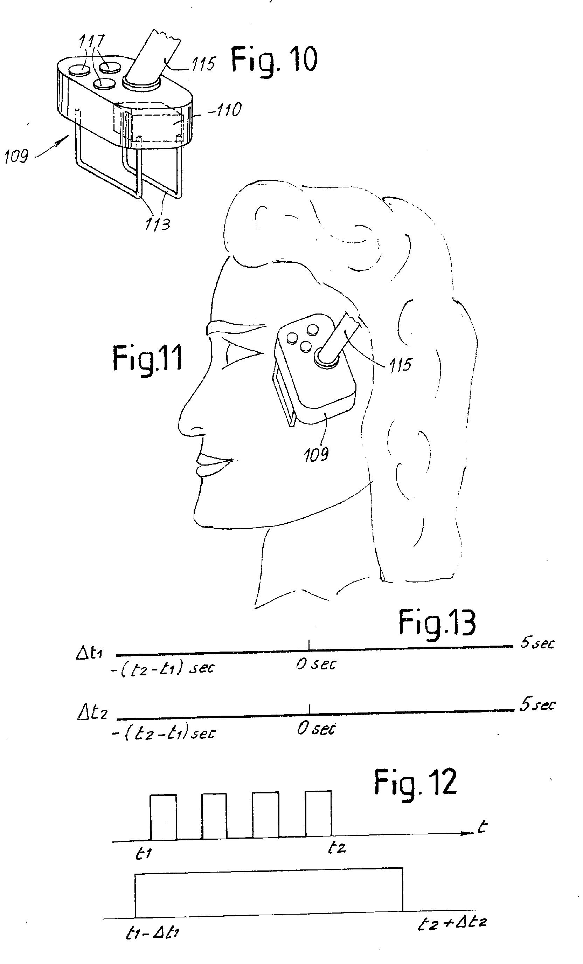

According to improved embodiments of the present invention, the laser treatment is combined with a treatment by means of radio frequency application. Figures 10 to 13 illustrate this embodiment. Figure 10 shows a handpiece 109, containing the same components as handpiece 9, in addition to a radio frequency generator, schematically illustrated with the number 110. The radio frequency generator is connected to a pair of electrodes 113. In some embodiments, the electrodes 113 are shaped so as to form a spacer between the handpiece 109 and the surface to be treated. The distance is set based upon the optical characteristics of the laser, whose radiation is conveyed to the handpiece 109 by means of a light guide 115 as in the previously described embodiment. On the handpiece 109 interface means are provided between the equipment and the user, such as for example one or more buttons or other, generically indicated with the number 117.

Using the electrodes as spacers, a particularly compact instrument is obtained, economical and easy to use.

With such a handpiece it is possible to combine in a synergic manner the effects of the laser and of the radio frequency on the treated tissues. More in particular, the radio frequency emission allows heating the tissues below the tissues exposed to the laser radiation, so that also the underlying collagen layers are subjected to heating, induced in this case by the radio frequency, and are subjected to a shrinking, which results in toning up the skin. When the electrodes 113 rest on the skin to be treated, for instance on the face of the patient, as shown in figure 11, the radio frequency field generated by the electrodes propagates in the tissues and generates induced currents, which heat the tissue of the derma at greater depths than the depths where the laser has effect.

The laser radiation and the radio frequency can be combined or temporarily overlapped in different manners. The diagram of figure 12 shows a time graph of the laser emission and of the radio frequency emission (RF). The pulsed laser radiation is emitted in the time interval between tl and t2. The radio frequency emission is applied in an interval tl - $\Delta$t1 and t2 + $\Delta$t2, wherein

$\Delta$t1 can vary for example between 10 seconds and - (t2 - t1)

$\Delta$t2 can vary for example between 10 seconds and - (t2 - t1)

as graphically represented in figure 13. By imposing the condition

(t1 - $\Delta$t1) < (t2 - $\Delta$t2)

it is possible to vary the starting time and the ending time of the radio frequency emission relative to the interval of laser treatment. The variation can be continuous or discontinuous. For example, it is possible to provide for a stepped regulation with steps of 0.01 seconds or less. In this way it is possible to start the radio frequency emissions ten seconds before the laser treatment or in a subsequent time, which can be temporarily translated until at most it coincides with the end time of the laser emission, thus making the two treatments in time sequence instead of in overlapping, starting with the laser radiation and then prosecuting with the RF radiation. The ending time of the RF radiation can be from ten seconds after the end of the laser emission or it can be advanced until to coincide with the instant corresponding to that in which the laser emission starts. Also in this case the two treatments are performed in sequence, firstly the radio frequency treatment and subsequently the laser treatment.

When $\Delta$t1=$\Delta$t2=0, the two treatments (laser and RF) are temporarily overlapped and have the same time duration.

Example of values, which can be used in the combined laser + radio frequency electric field application, are the following:

- spacing between the pulses: 1 mm
- laser power: 30W
- RF power: 10W
- energy per pulse: 50 mJ

In general, the space between the pulses can vary between 0.01 and 5 mm and preferably between 0.1 and 3 mm. The laser power can vary between 0.5 and 70W, preferably between 2 and 50 W and more preferably between 10 and 40 W. The power of the radio frequency field can be comprised between 0.1 and 30W, preferably between 1 and 20 W and more preferably between 4 and 18 W. Lastly, the energy per pulse of the laser beam can be comprised between 1 mJ and 200 mJ, preferably between 10 mJ and 100 mJ and more preferably between 20 mJ and 100 mJ. It should be understood that the data indicated above are given exclusively by way of non limiting example and that it is possible to use also data outside these intervals. Furthermore, also intermediate intervals between that indicated are comprised in the present description, being understood that the present description relates to any intermediate value and to any intermediate interval between the indicated values.

It is understood that the drawing only shows an example provided by way of a practical demonstration of the present invention, which can vary in forms and arrangements without however departing from the scope of the concept underlying the invention. Any reference numbers in the appended claims are provided to facilitate reading of the claims with reference to the description and to the drawing, and do not limit the scope of protection represented by the claims.

**Claims**

1.  A device (1)
    for laser treatment of the epidermis comprising:

    - a laser energy source; (5)
    - a laser energy focusing system, arranged and controlled to focus a laser beam on a plurality of contiguous volumes of a region of the epidermis;
    - a scanning and actuating system to address a laser beam on a plurality of portions of a region of epidermis to be treated, controlled so as to actuate a laser beam in a plurality of positions inside said region of epidermis to be treated;

    wherein: said laser beam has a variable energy density profile in a cross section of said beam; wherein the energy density profile of the laser beam has approximately a shape of a Gaussian curve, with the maximum arranged on the axis of the beam; in a central area of said cross section the intensity of the beam is suitable to cause an ablation of the epidermis in a central portion of the volume exposed to the laser beam; in an intermediate annular area of said cross section the intensity of the beam is suitable to cause hemostasis of the blood vessels and shrinking of the collagen of the epidermis in an annular portion of the volume exposed to the laser beam, said annular portion surrounding said central portion; in an area outside the intermediate annular area the intensity of the beam is suitable to cause a biostimulation of the tissues; and further comprising a control of the emission of the beam and of the focusing system which emits a plurality of laser pulses for each of a series of positions sequentially taken by the laser beam; wherein said central area has a maximum cross dimension from about 40 to about 500 micrometers; and wherein said intermediate annular area has an inner dimension corresponding to the dimension of the central area and a maximum external cross dimension from 6 to 200 micrometers greater than the cross dimension of the central area.

2.  A device as claimed in claim 1, wherein said laser energy focusing system is arranged and controlled so as to treat contiguous volumes of the epidermis arranged according to a pattern, wherein each treated volume has a center substantially arranged on the axis of the laser beam used for treating said volume, the axes of the laser beams used to treat said contiguous volumes being distributed according to a presettable dot matrix.

3.  A device as claimed in claim 2, wherein the energy density profile of the laser beam to treat said contiguous volumes and the distances between dots of said presettable matrix are such that all the treated region of the epidermis is subjected to the action of the laser energy.

4.  A device as claimed in one or more of the previous claims, wherein said central area has a maximum cross dimension from about 100 to about 400 micrometers and preferably from about 120 to about 350 micrometers.

5.  A device as claimed in one or more of the previous claims, wherein said intermediate annular area has an inner dimension corresponding to the dimension of the central area and a maximum external cross dimension from 80 to 120 micrometers greater than the cross dimension of the central area.

6.  A device as claimed in one or more of the previous claims, wherein said laser energy source has a wave length comprised between 532 and 13,000 nm.

7.  A device as claimed in claim 6, wherein said laser energy source is a $CO_2$ laser with a 10600 nm emission.

8.  A device as claimed in one or more of the previous claims, comprising a scanning head to apply said laser energy by means of a laser beam scanned and actuated in an intermitting manner in correspondence of said volumes.

9. 10. A device as claimed in claim 9, comprising a wave guide to convey the laser energy towards an applying handpiece, inside which said scanning head is housed.

10. A device as claimed in one or more of the previous claims, comprising a radio frequency generator and at least one electrode arranged on a handpiece, said handpiece being connected to said laser energy source by means of a wave guide.

11. A device as claimed in claim 11, wherein said radio frequency generator is housed inside said handpiece.

12. A device as claimed in claim 11 or 12, wherein said at least one electrode forms a spacer between said handpiece and the tissue to be treated.

13. A device as claimed in claim 11, 12 or 13, comprising two electrodes, carried by said handpiece, for propagating the radio frequency emission.

14. A device as claimed in one or more of claims 12 to 14, comprising a time control, for temporarily coordinating the application of the laser energy and of the radio frequency emission.

15. A device as claimed in claim 15, wherein said time control allows performing an at least partially overlapped application, or an application in sequence and without overlapping of the laser energy and of the radio frequency emission.


**Patentansprüche**

1. Vorrichtung (1) zur Laserbehandlung der Epidermis mit:

    - einer Laserenergie-Quelle (5),
    - einem Laserenergie-Fokussierungssystem, das angeordnet und gesteuert ist, um einen Laserstrahl auf eine Anzahl von zusammenhängenden Volumina eines Bereichs der Epidermis zu fokussieren,
    - einem Abtast- und Betätigungssystem, um einen Laserstrahl auf eine Anzahl von Teilen eines Bereichs der zu behandelnden Epidermis zu richten, das so gesteuert ist, um einen Laserstrahl in einer Anzahl von Positionen innerhalb des Bereichs der zu behandelnden Epidermis zu betätigen,

    wobei: der Laserstrahl ein variables Energiedichte-Profil in einem Querschnitt des Strahls aufweist, wobei das Energiedichte-Profil des Laserstrahls annähernd eine Form einer Gauß'schen Kurve aufweist, wobei das Maximum auf der Achse des Strahls angeordnet ist, wobei in einem zentralen Bereich des Querschnitts die Intensität des Strahls geeignet ist, um eine Abtragung der Epidermis in einem zentralen Bereich des Volumens, das dem Laserstrahl ausgesetzt ist, zu bewirken, wobei in einem ringförmigen Zwischenbereich des Querschnitts die Intensität des Strahls geeignet ist, um eine Hämostase der Blutgefäße und ein Schrumpfen des Kollagens der Epidermis in einem ringförmigen Bereich des Volumens, das dem Laserstrahl ausgesetzt ist, zu bewirken, wobei der ringförmige Bereich den zentralen Bereich umgibt, wobei in einem Bereich außerhalb des ringförmigen Zwischenbereich die Intensität des Strahls geeignet ist, um eine Biostimulation des Gewebes zu verursachen, und mit ferner einer Steuerung der Emission des Strahls und des Fokussierungssystems, das eine Anzahl von Laserpulsen für jeweils eine Folge von Positionen emittiert, die sequenziell von dem Laserstrahl eingenommen werden, wobei der zentrale Bereich eine maximale Querschnittsabmessung von etwa 40 bis etwa 500 $\mu$m aufweist und wobei der ringförmige Zwischenbereich eine innere Abmessung entsprechend der Abmessung des zentralen Bereichs und eine maximale äußere Querschnittsabmessung von 6 bis 200 $\mu$m mehr als die Querschnittsabmessung des zentralen Bereichs aufweist.

2. Vorrichtung nach Anspruch 1, wobei das Laserenergie-Fokussierungssystem so angeordnet und gesteuert ist, dass es zusammenhängende Volumina der Epidermis behandelt, die gemäß einem Muster angeordnet sind, wobei jedes behandelte Volumen eine Mitte aufweist, die im Wesentlichen auf der Achse des Laserstrahls angeordnet ist, der zur Behandlung des Volumens verwendet wird, wobei die Achsen der Laserstrahlen, die zur Behandlung der zusammenhängenden Volumina verwendet werden, entsprechend einer voreinstellbaren Punktmatrix verteilt sind.

3. Vorrichtung nach Anspruch 2, wobei das Energiedichte-Profil des Laserstrahls, um die zusammenhängenden Volumina zu behandeln, und die Abstände zwischen den Punkten der voreinstellbaren Matrix so sind, dass alle behandelten Bereiche der Epidermis der Aktion der Laserenergie ausgesetzt sind.

**4.** Vorrichtung nach einem oder mehreren der vorstehenden Ansprüche, wobei der zentrale Bereich eine maximale Querschnittsabmessung von etwa 100 bis etwa 400 $\mu$m und vorzugsweise von etwa 120 bis etwa 350 $\mu$m aufweist.

**5.** Vorrichtung nach einem oder mehreren der vorstehenden Ansprüche, wobei der ringförmige Zwischenbereich eine innere Abmessung entsprechend der Abmessung des zentralen Bereichs und eine maximale externe Querschnittsabmessung von 80 bis 120 $\mu$m größer als die Querschnittsabmessung des zentralen Bereichs aufweist.

**6.** Vorrichtung nach einem oder mehreren der vorstehenden Ansprüche, wobei die Laserenergie-Quelle eine Wellenlänge zwischen 532 und 13.000 nm aufweist.

**7.** Vorrichtung nach Anspruch 6, wobei die Laserenergie-Quelle ein $CO_2$-Laser mit einer Emission bei 10.600 nm ist.

**8.** Vorrichtung nach einem oder mehreren der vorstehenden Ansprüche mit einem Abtastkopf zur Anwendung der Laserenergie mittels eines Laserstrahls, der in intermittierender Weise entsprechend den Volumina geführt und betätigt wird.

**9.** Vorrichtung nach Anspruch 9 mit einem Wellenleiter zur Führung der Laserenergie zu einem Anwendungs-Handgriff, innerhalb dessen der Abtastkopf aufgenommen ist.

**10.** Vorrichtung nach einem oder mehreren der vorstehenden Ansprüche mit einem Funkfrequenz-Generator und mindestens einer Elektrode, die an einem Handgriff angeordnet ist, wobei der Handgriff mittels eines Wellenleiters mit der Laserenergie-Quelle verbunden ist.

**11.** Vorrichtung nach Anspruch 11, wobei der Funkfrequenz-Generator innerhalb des Handgriffs aufgenommen ist.

**12.** Vorrichtung nach Anspruch 11 oder 12, wobei die mindestens eine Elektrode einen Abstandshalter zwischen dem Handgriff und dem zu behandelnden Gewebe bildet.

**13.** Vorrichtung nach Anspruch 11, 12 oder 13 mit zwei Elektroden, die von dem Handgriff getragen werden, um die Funkfrequenz-Emission weiterzuleiten.

**14.** Vorrichtung nach einem der Ansprüche 12 bis 14 mit einer Zeitsteuerung zum zeitweiligen Koordinieren der Anwendung der Laserenergie und der Funkfrequenz-Emission.

**15.** Vorrichtung nach Anspruch 15, wobei die Zeitsteuerung die Durchführung einer zumindest teilweise überlappenden Anwendung oder einer Anwendung nacheinander und ohne Überlappung der Laserenergie und der Funkfrequenz-Emission ermöglicht.

**Revendications**

**1.** Un dispositif (1) pour le traitement au laser de l'épiderme, comprenant :

   - une source d'énergie laser (5) ;
   - un système de focalisation de l'énergie laser, agencé et commandé pour focaliser un faisceau laser sur une pluralité de volumes contagieux d'une zone de l'épiderme ;
   - un système de balayage et d'actionnement pour diriger un faisceau laser sur une pluralité de parties d'une zone d'épiderme à traiter, commandé de manière à actionner un faisceau laser dans une pluralité de positions à l'intérieur de ladite zone d'épiderme à traiter ; dans lequel : ledit faisceau laser a un profil de densité d'énergie variable dans une section transversale dudit faisceau laser ; dans lequel le profil de densité d'énergie du faisceau laser a approximativement la forme d'une courbe de Gausse, avec le maximum agencé sur l'axe du faisceau ; dans une zone centrale de ladite section transversale, l'intensité du faisceau est apte à provoquer une ablation de l'épiderme dans une partie centrale du volume exposée au faisceau laser ; dans une surface annulaire intermédiaire de ladite section transversale, l'intensité du faisceau est apte à provoquer une hémostase des vaisseaux sanguins et un rétrécissement du collagène de l'épiderme dans une partie annulaire du volume exposée au faisceau laser, ladite partie annulaire entourant ladite partie centrale ; dans une surface à l'extérieur de la surface annulaire intermédiaire, l'intensité du faisceau est apte à provoquer une bio-stimulation des tissus ; et comprenant en outre une commande de l'émission du faisceau et du système de focalisation qui émet une pluralité de pulsations laser pour chacune d'une série de positions prises séquentiellement par le faisceau laser ; dans lequel ladite surface centrale a une dimension transversale maximale allant d'environ 40 à environ 500 micromètres ; et dans lequel ladite surface annulaire intermédiaire a une dimension interne correspondant à la dimension de la surface centrale et une dimension transversale externe maximale supérieure de 6 à 200 micromètres à la dimension transversale de la surface centrale.

2. Un dispositif selon la revendication 1, dans lequel ledit système de focalisation d'énergie laser est agencé et commandé de manière à traiter des volumes contigus de l'épiderme agencés suivant un motif, dans lequel chaque volume traité a un centre agencé sensiblement sur l'axe du faisceau laser utilisé pour traiter ledit volume, les axes des faisceaux lasers utilisés pour traiter lesdits volumes contigus étant distribués suivant une matrice de points prédéterminable.

3. Un dispositif selon la revendication 2, dans lequel le profil de densité d'énergie du faisceau laser pour traiter lesdits volumes contigus et les distances entre les points de ladite matrice prédéterminable sont telles que toute la zone traitée de l'épiderme est soumise à l'action de l'énergie laser.

4. Un dispositif selon l'une ou plusieurs des revendications précédentes, dans lequel ladite surface centrale a une dimension transversale maximale d'environ 100 à environ 400 micromètres et de préférence d'environ 120 à environ 350 micromètres.

5. Un dispositif selon l'une ou plusieurs des revendications précédentes, dans lequel ladite surface annulaire intermédiaire a une dimension interne correspondant à la dimension de la surface centrale et une dimension transversale externe maximale supérieure de 80 à 120 micromètres à la dimension transversale de la surface centrale.

6. Un dispositif selon l'une ou plusieurs des revendications précédentes, dans lequel ladite source d'énergie laser a une longueur d'onde comprise entre 532 et 13 000 nm.

7. Un dispositif selon la revendication 6, dans lequel ladite source d'énergie laser est un laser au $CO_2$ ayant une émission de 10 600 nm.

8. Un dispositif selon l'une ou plusieurs des revendications précédentes, comprenant un tête de balayage pour appliquer ladite énergie laser au moyen d'un faisceau laser balayé et actionné de manière intermittente en correspondance avec lesdits volumes.

9. Un dispositif selon la revendication 8, comprenant un guide d'ondes pour diriger l'énergie laser vers une pièce à main d'application, à l'intérieur de laquelle ladite tête de balayage est logée.

10. Un dispositif selon l'une ou plusieurs des revendications précédentes, comprenant un générateur de fréquences radio et au moins une électrode agencée sur une pièce à main, ladite pièce à main étant connectée à ladite source d'énergie laser au moyen d'un guide d'ondes.

11. Un dispositif selon la revendication 10, dans lequel ledit générateur de fréquence radio est logé à l'intérieur de ladite pièce à main.

12. Un dispositif selon la revendication 10 ou 11, dans lequel ladite ou lesdites électrode(s) forme(nt) une entretoise entre ladite pièce à main et le tissu à traiter.

13. Un dispositif selon la revendication 10, 11 ou 12, comprenant deux électrodes, portées par ladite pièce à main, pour propager l'émission de fréquences radio.

14. Un dispositif selon l'une ou plusieurs des revendications 10 à 13, comprenant une commande de temps, pour coordonner temporairement l'application de l'énergie laser et de l'émission de fréquences radio.

15. Un dispositif selon la revendication 14, dans lequel ladite commande de temps permet la réalisation d'une application au moins partiellement superposée, ou d'une application en séquence et sans superposition de l'énergie laser et de l'émission de fréquences radio.

Fig.1

Fig.2

Fig. 3

Fig. 4

Fig. 5

Fig.6

(b) (b) (b) (b)

(c)

(a) (a)

Fig.7

(b) (b)

(a) (a)

Fig.8

25

23

21

21

23

F

Fig.9

F

Fig. 10

117
115
110
109
113

Fig.11

115
109

Fig.13

$\Delta t_1$ ———————————————— 5 sec
$-(t_2-t_1)$ sec        0 sec

$\Delta t_2$ ———————————————— 5 sec
$-(t_2-t_1)$ sec        0 sec

Fig. 12

t

$t_1$        $t_2$

$t_1-\Delta t_1$        $t_2+\Delta t_2$

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6997923 B
- WO 2006116141 A
- WO 2008128175 A
- WO 2005007003 A
- WO 2007095183 A

**Non-patent literature cited in the description**

- **CHERNOFF G ; SLATKINE M ; ZAIR E ; MEAD D.** SilkTouch: a new technology for skin resurfacing in aesthetic surgery. *J Clin Laser Med Surg.,* April 1995, vol. 13 (2), 97-100
- **WALDORF HA ; KAUVAR AN ; GERONEMUS RG.** Skin resurfacing of fine to deep rhytides using a char-free carbon dioxide laser in 47 patients. *Dermatol Surg.,* November 1995, vol. 21 (11), 940-6
- **DAVID LM ; SAME AJ ; UNGER WP.** Rapid laser scanning for facial resurfacing. *Dermatol Surg.,* December 1995, vol. 21 (12), 1031-3
- **LASK G ; KELLER G ; LOWE N ; GORMLEY D.** Laser skin resurfacing with the SilkTouch flashscanner for facial rhytides. *Dermatol Surg.,* December 1995, vol. 21 (12), 1021-4
- **APFELBERG DB.** Ultrapulse carbon dioxide laser with CPG scanner for full-face resurfacing for rhytids, photoaging, and acne scars. *Plast Reconstr Surg.,* June 1997, vol. 99 (7), 1817-25
- **APFELBERG DB ; SMOLLER B.** UltraPulse carbon dioxide laser with CPG scanner for deepithelialization: clinical and histologic study. *Plast Reconstr Surg.,* June 1997, vol. 99 (7), 2089-94
- **RAULIN C ; DROMMER RB ; SCHÖNERMARK MP ; WERNER S.** Facial wrinkles--ultrapulsed CO2 laser: alternative or supplement to surgical face lift?. *Laryngorhinootologie,* June 1997, vol. 76 (6), 351-7
- **TRELLES MA ; RIGAU J ; MELLOR TK ; GARDA L.** A clinical and histological comparison of flashscanning versus pulsed technology in carbon dioxide laser facial skin resurfacing. *Dermatol Surg.,* January 1998, vol. 24 (1), 43-9
- **WEINSTEIN C.** Computerized scanning erbium:YAG laser for skin resurfacing. *Dermatol Surg.,* January 1998, vol. 24 (1), 83-9
- **BERNSTEIN LJ ; KAUVAR AN ; GROSSMAN MC ; GERONEMUS RG.** Scar resurfacing with high-energy, short-pulsed and flashscanning carbon dioxide lasers. *Dermatol Surg.,* January 1998, vol. 24 (1), 101-7
- **VAÏSSE V ; CLERICI T ; FUSADE T.** Bowen disease treated with scanned pulsed high energy CO2 laser. Follow-up of 6 cases. *Ann. Dermatol. Venereol.,* November 2001, vol. 128 (11), 1220-4
- Laser Dermatology - State of the Art. **TOSHIO OHSHIRO et al.** proceedings of the 7th Congress International Society for Laser Surgery and Medicine in Connection with Laser 87 Optoelectronics. 1988, 513 ff
- **FITZPATRLCK RE ; ROSTAN EF ; MARCHELL N.** Collagen tightening induced by carbon dioxide laser versus erbium: YAG laser. *Lasers Surg. Med.,* 2000, vol. 27 (5), 395-403
- **HASEGAWA T ; MATSUKURA T ; MIZUNO Y ; SUGA Y ; OGAWA H ; IK:EDA S.** Clinical trial of a laser device called fractional photothermolysis system for acne scars. *Dermatol.,* September 2006, vol. 33 (9), 623-7
- **RAHMAN Z ; ALAM M ; DOVER JS.** Fractional Laser treatment for pigmentation and texture improvement. *Skin Therapy Lett.,* November 2006, vol. 11 (9), 7-11
- **LAUBACH H ; CHAN HH ; RIUS F ; ANDERSON RR ; MANSTEIN D.** Effects of skin temperature on lesion size in fractional photothermolysis. *Lasers Surg Med.,* January 2007, vol. 39 (1), 14-8
- **COLLAWN SS.** Fraxel skin resurfacing. *Ann Plast Surg.,* March 2007, vol. 58 (3), 237-40
- **HANTASH BM ; BEDI VP ; CHAN KF ; ZACHARY CB.** Ex vivo histological characterization of a novel ablative fractional resurfacing device. *Lasers Surg Med.,* February 2007, vol. 39 (2), 87-95
- **HANTASH BM ; BEDI VP ; KAPADIA B ; RAHMAN Z ; JIANG K ; TANNER H ; CHAN KF.** In vivo histological evaluation of a novel ablative fractional resurfacing device. *Lasers Surg Med.,* February 2007, vol. 39 (2), 96-107
- **GOLDBERG DJ ; FAZELI A ; BERLIN AL.** Clinical, laboratory, and MR1 analysis of cellulite treatment with a unipolar radiofrequency device. *Dermatol Surg.,* February 2008, vol. 34 (2), 204-9

- **MONTESI G ; CALVI ERI S ; BALZANI A ; GOLD MH.** Bipolar radiofrequency in the treatment of dermatologic imperfections: clinicopathological and immunohistochemical aspects. *J.Drugs Dermatol.,* February 2007, vol. 6 (2), 212-5